# EUROPÄISCHE PATENTSCHRIFT

(11) **EP 2 392 642 B1**
(45) Veröffentlichungstag und Bekanntmachung des Hinweises auf die Patenterteilung: **10.09.2014**
(21) Anmeldenummer: 11168292.8
(22) Anmeldetag: 31.05.2011
(51) Int. Cl.: C12M 1/107, A01C 3/02, B01F 5/10, B01F 7/00, B01F 5/06

(54) **Verfahren zur Biogaserzeugung**
Method for producing biogas
Procédé destiné à produire du biogaz

(30) Priorität: 01.06.2010 AT 36110 U
(43) Veröffentlichungstag der Anmeldung: 07.12.2011
(73) Patentinhaber: Hörmann Interstall GmbH & Co. KG, 3352 St. Peter/Au (AT)
(72) Erfinder: Thaler, Albert, 4030 Linz (AT); Schweitzer, Franz, 4074 Stroheim (AT); Führer, Franz, 3631 Ottenschlag (AT)
(74) Vertreter: KLIMENT & HENHAPEL

(56) Entgegenhaltungen:
- EP-A1- 0 466 946
- EP-A1- 1 985 692
- DE-A1- 3 938 248
- DE-A1-102005 061 039

## Beschreibung

### GEBIET DER ERFINDUNG

Verfahren zur Erzeugung von Biogas durch anaerobe Vergärung organischer Einsatzstoffe, welche in flüssiger und/oder fester Form in einen vorzugsweise beheizbaren Gärbehälter eingebracht werden und in weiterer Folge in einen geschlossenen Nachgärbehälter bzw. ein Endlager für die Zwecke des weiteren Ausgärens umgelagert werden.

Die vorliegende Erfindung bezieht sich weiters auf einen gasdicht abgeschlossenen, vorzugsweise beheizbaren Gärbehälter zum Einsatz in einem Verfahren zur Erzeugung von Biogas durch anaerobe Vergärung organischer Einsatzstoffe, bei welchem die organischen Einsatzstoffe in flüssiger und/oder fester Form in den Gärbehälter eingebracht werden und in weiterer Folge in einen geschlossenen Nachgärbehälter bzw. ein Endlager für die Zwecke des weiteren Ausgärens umgelagert werden.

### STAND DER TECHNIK

Verfahren zur Herstellung von Biogas mittels Vergärung von organischen Einsatzstoffen sind bekannt. Die Einsatzstoffe, beispielsweise Silage (Gras, Mais, Hafer, Gerste, Weizen, Hirse, Energiepflanzen), biogene Abfälle (Gemüse, Grünschnitt), Press- und Gärrückstände, Pflanzenöl (aus Fettabscheidern), Schlachtabfälle, CCM (Corn-Crops-Mix) sowie prinzipiell alle vergärbaren biogenen Substrate werden in fester oder flüssiger Form in einen Gärbehälter, auch Fermenter genannt, eingebracht und mittels Rührwerken in Zeittakten durchmischt. Das dabei entstehende Gärgas wird durch geeignete Mittel gesammelt und entweder als Primärenergieträger gespeichert oder unmittelbar einer weiteren Verwendung zugeführt, wie beispielsweise der Verbrennung in einem Blockheizkraftwerk oder in einer Gasturbine (Wärme und Strom), in einem Biogasheizkessel (Wärme), der Verwendung als Treibstoff (CNG - Compressed Natural Gas) oder der Einspeisung in das Erdgasnetz.

In der Regel werden im Anschluss an die zumindest teilweise Entgasung der Einsatzstoffe im Gärbehälter diese für die Zwecke des weiteren Ausgärens in einen geschlossenen Nachgärbehälter bzw. in ein (offenes) Endlager befördert.

Die Durchmischung im Gärbehälter ist erforderlich, um die Bildung von Sinkschichten sowie Schwimmschichten an der Oberfläche, die in der Regel aus Fetten und freien Fettsäuren bestehen und beide eine ordnungsgemäße Entgasung verhindern, zu vermeiden.

Bei den zum Einsatz kommenden Rührwerken handelt es sich meist um Tauchmotor-Propellerrührwerke, Stabmixer, mit außerhalb des Gärbehälters angeordneten Motoren, Langachsrührwerke oder Paddelrührwerke, die an mehreren Stellen im Gärbehälter angeordnet sind. Sie benötigen den Großteil der für den Anlagenbetrieb erforderlichen Energie und sind konstruktionsbedingt ausschließlich zur Durchmischung der im Gärbehälter befindlichen Einsatzstoffe geeignet. Je kleiner die zum Einsatz kommenden Rührwerke sind, desto lokal begrenzter ist ihre Mischwirkung, je größer, desto teurer in der Anschaffung und aufwändiger in der Konstruktion und höher der Energiebedarf.

Ein solcher Gärbehälter samt Rührwerk ist beispielsweise aus der DE 10 2005 061039 A1 bekannt, wobei dort die Erhaltung einer Schwimmschicht als Siedlungsfläche für Bakterien zunächst ausdrücklich erwünscht ist.

Um jedoch eine einwandfreie Durchmischung der Einsatzstoffe zu ermöglichen und die Bildung der Sink- und Schwimmschichten wirkungsvoll zu verhindern, kommen stets mehrere Rührwerke zum Einsatz, die an unterschiedlichen vertikalen Positionen im Gärbehälter oder aber tiefenverstellbar angeordnet sind, deren Einsatz jedoch aufgrund der Anzahl sehr energieintensiv ist.

Ein weiteres Problem bei bekannten, bereits ausgeführten Anlagen zur Erzeugung von Biogas ist die Einbringung von frischen Einsatzstoffen in den Gärbehälter. Dazu ist es üblich, die frischen Einsatzstoffe in flüssiger oder angemaischter flüssiger/fester Form entweder mittels Pumpen oder mittels einer annähernd vertikal angeordneten Einbringschnecke (in fester Form) in den Gärbehälter zu befördern und die frischen Einsatzstoffe dann durch in diesem Einbringbereich installierte Rührwerke möglichst gleichmäßig in das Substratvolumen im Gärbehälter einzumischen. Dennoch kommt es oft zu einer ungleichmäßigen Verteilung der frischen Einsatzstoffe und damit zu einer erhöhten Gefahr der Ausbildung von Sink- und Schwimmschichten.

Aus der Gülletechnik ist es bekannt, die in der Güllegrube lagernde Gülle zwangsumzuwälzen. Ein Vergärungsprozess kommt hier nicht in Gang, da die Gülle hier nicht unter Luftabschluss (anaerob) sondern im aeroben Bereich und bei einer Temperatur von weniger als 20°C gelagert wird. Dabei ist in der Güllegrube durch Güllegrubenboden, Güllegrubendeckel und Zwischenwände ein Ringkanalsystem ausgebildet, welches im wesentlichen einen einheitlichen Strömungsquerschnitt aufweist. An einer beliebigen Position innerhalb des Ringkanalsystems ist eine den Strömungsquerschnitt an dieser Position komplett ausfüllende Schottwand angeordnet, welche von einer in seiner vertikalen Position im Güllebehälter variierbaren Öffnung durchsetzt ist, deren Querschnitt geringer als jener des Ringkanalsystems ist. Mit dieser Öffnung ist ein Strömungsring/-rohr gekoppelt, in welchem ein Rotor eines Rührwerks angeordnet ist. Im Betrieb saugt das Rührwerk Gülle in das Strömungsring/-rohr und befördert es strahlartig durch die Öffnung auf die gegenüberliegende Seite der Schottwand.

Durch die dabei erzwungene Umwälzung und gleichzeitige Zerkleinerung von langstieligen festen Bestandteilen (beispielsweise Strohhalmen) der Gülle wird eine gleichmäßige Beschaffenheit derselben erreicht, was für die spätere Verteilung auf Feldern wichtig ist.

### AUFGABE DER ERFINDUNG

Die Aufgabe der vorliegenden Erfindung ist es, den Energieeinsatz bei der Biogaserzeugung zu verringern und gleichzeitig die Bildung von Sink- und Schwimmschichten wirkungsvoll zu verhindern, um eine einwandfreie Entgasung zu ermöglichen.

Eine weitere Aufgabe der Erfindung ist es, die Einbringung von frischen Einsatzstoffen zu erleichtern und eine rasche Homogenisierung des gesamten Substratvolumens zu ermöglichen.

### DARSTELLUNG DER ERFINDUNG

Erfindungsgemäß wird dies dadurch erreicht, dass beim anaerob ablaufenden Prozess der Biogaserzeugung ein nahezu identisches System eingesetzt wird, wie im aerob ablaufenden Prozess der Gülletechnik.

Konkret ist es bei einem Verfahren zur Erzeugung von Biogas durch anaerobe Vergärung organischer Einsatzstoffe, welche in flüssiger und/oder fester Form in einen beheizbaren Gärbehälter eingebracht werden und in weiterer Folge in einen geschlossenen Nachgärbehälter bzw. Endlager oder in ein offenes Endlager für die Zwecke des weiteren Ausgärens umgelagert werden, vorgesehen, dass die organischen Einsatzstoffe in einem einen mittleren Strömungsquerschnitt aufweisenden Ringkanalsystem des Gärbehälters umgewälzt werden, wobei die Umwälzung durch eine Antriebseinheit bewirkt wird, welche die organischen Einsatzstoffe durch einen gegenüber dem mittleren Strömungsquerschnitt des Ringkanalsystems verringerten, vorzugsweise betreffend seine vertikale Position im Gärbehälter einstellbaren, Umwälzquerschnitt einer Öffnung befördert, welche in einer einen Querschnitt des Ringkanalsystems ausfüllenden Schottwand angeordnet ist, welche unterhalb und oberhalb der Öffnung aus faltbaren, in ihrer Länge variablen Schottvorhängen besteht.

Der Begriff vertikal ist dabei in Bezug auf den Betriebszustand des Gärbehälters zu verstehen.

Überraschenderweise hat sich nämlich herausgestellt, dass durch die Zwangsumwälzung durch einen definierten Umwälzquerschnitt im Ringkanalsystem, welcher Umwälzquerschnitt kleiner ist als der mittlere Strömungsquerschnitt des Ringkanalsystems, neben der bereits aus der Gülletechnik bekannten guten Durchmischung der Einsatzstoffe auch die Sink- und Schwimmschichtbildung, eine Problematik die in der Gülletechnik gar nicht auftritt, so zuverlässig verhindert werden kann, dass der Einsatz von zusätzlichen Rührwerken dadurch obsolet wird.

Anstelle der bei der Erzeugung von Biogas sonst üblichen Durchmischung der Einsatzstoffe mittels Rührwerken an mehreren Stellen innerhalb des Gärbehälters, ist es erfindungsgemäß nunmehr möglich, die Einsatzstoffe durch Umwälzen an wenigen Stellen bzw. in den meisten Fällen sogar nur an einer einzigen Stelle im Gärbehälter derart in Bewegung zu halten / zu zirkulieren, dass sich keine Sink- und Schwimmschichten ausbilden können.

Zusätzlich hat sich herausgestellt, dass durch das Einbringen von frischen Einsatzstoffen unmittelbar im Ansaugbereich der Antriebseinheit und die darauf folgende sofortige Durchführung durch den Umwälzquerschnitt eine besonders gleichmäßige Verteilung, der frischen Einsatzstoffe bewirkt und die festen Einsatzstoffe dadurch kontinuierlich in Schwebe gehalten werden, wodurch die Entgasung optimiert werden kann.

In einer bevorzugten Ausführungsvariante der Erfindung erfolgt die Umwälzung durch den Umwälzquerschnitt in Zeittakten.

Neben dem erfindungsgemäßen Verfahren, welches zur Lösung der der Erfindung zugrundeliegenden Aufgabe vorgeschlagen wird, ist auch ein Gärbehälter zur Durchführung des Verfahrens zur Lösung der der Erfindung zugrundeliegenden Aufgabe vorgesehen.

Dieser ist gasdicht abgeschlossenen ausgeführt und vorzugsweise beheizbar und mit Mittel zur Entnahme von durch die Gärung entstehendem Biogas ausgestattet und weist erfindungsgemäß eine Antriebseinheit zur Umwälzung der organischen Einsatzstoffe in einem Ringkanalsystem des Gärbehälters auf, wobei der Rotor der Antriebseinheit von einem Strömungsring/-rohr umgeben ist, welches an eine Öffnung einer einen Querschnitt des Ringkanalsystems ausfüllenden Schottwand angekoppelt ist und die Öffnung einen Umwälzquerschnitt aufweist, der kleiner ist als ein mittlerer Strömungsquerschnitt des Ringkanalsystems, wobei die Öffnung entlang der Schottwand vertikal verschiebbar ist und die Schottwand oberhalb und unterhalb der Öffnung aus faltbaren, in ihrer Länge variablen Schottvorhängen besteht.

Die Anordnung des Rotors der Antriebseinheit in einem Strömungsring/-rohr ermöglicht zusätzlich die Zerkleinerung der festen Einsatzstoffe.

In einer weiteren bevorzugten Ausführungsvariante der Erfindung ist es vorgesehen, dass das Ringkanalsystem meanderförmig ausgebildet ist. Dadurch erfolgt einerseits bei jeder Umlenkung der Strömung eine Wirbelbildung im in Bewegung befindlichen Substratvolumen was eine zusätzliche Durchmischung bedeutet und dadurch eine erleichterte Ausgasung, andererseits ist durch die kompakte Bauweise eine effektivere Beheizung durch Minimierung der Wärmeverlustflächen im Verhältnis zum Substratvolumen gegeben.

Ein besonders energieeffizienter Gärbehälter wird dadurch ermöglicht, dass im Gärbehälter lediglich eine einzige Schottwand samt Antriebseinheit vorgesehen ist. Im Gegensatz zu Gärbehältern herkömmlicher Anlagen zur Erzeugung von Biogas kann in dieser bevorzugten Ausführungsvariante auf den Einsatz mehrerer Rührwerke verzichtet werden, da aufgrund der Zwangsumwälzung im Ringkanalsystem der gesamte Inhalt des Ringkanalsystems zirkulieren kann und dabei die weiter oben genannten Effekte auftreten.

### KURZE BESCHREIBUNG DER FIGUREN

Die Erfindung wird nun anhand eines Ausführungsbeispiels näher erläutert. Die Zeichnungen sind beispielhaft und sollen den Erfindungsgedanken zwar darlegen, ihn aber keinesfalls einengen oder gar abschließend wiedergeben.

Dabei zeigt:
- Fig.1: eine schematische Darstellung eines erfindungsgemäßen Gärbehälters im Grundriss (Schnitt AA aus Fig.2)
- Fig.2: eine schematische Darstellung eines erfindungsgemäßen Gärbehälters im Seitenriss
- Fig.3: eine Ansicht der Schottwand entgegen der Umwälzrichtung (Schnitt CC aus Fig.1)
- Fig.4: eine Ansicht der Schottwand in Richtung der Umwälzrichtung (Schnitt DD aus Fig.1)
- Fig.5: ein Vertikalschnitt durch die Schottwand (Schnitt BB aus Fig.1)

### WEGE ZUR AUSFÜHRUNG DER ERFINDUNG

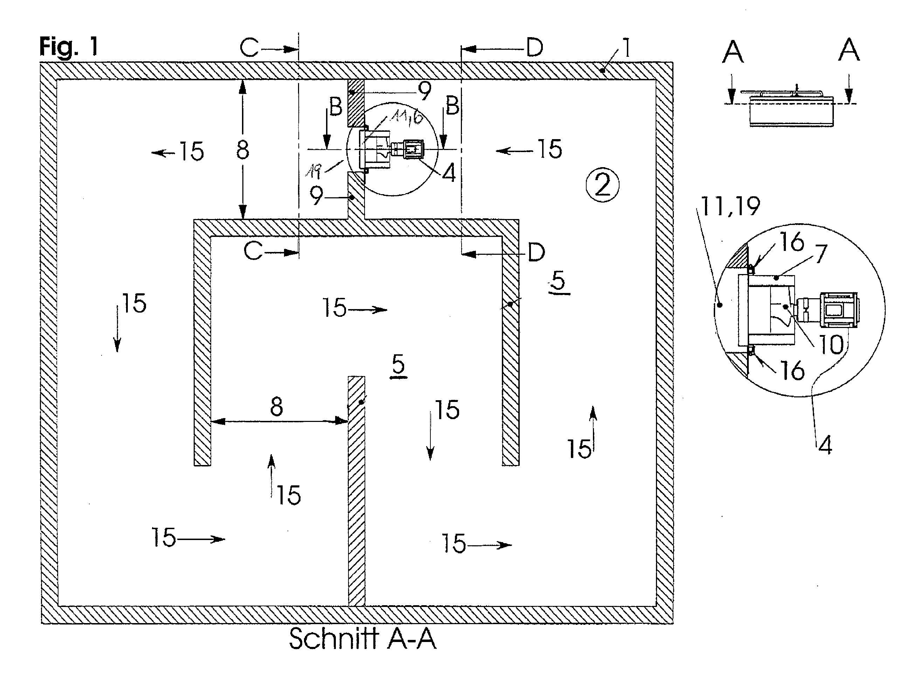

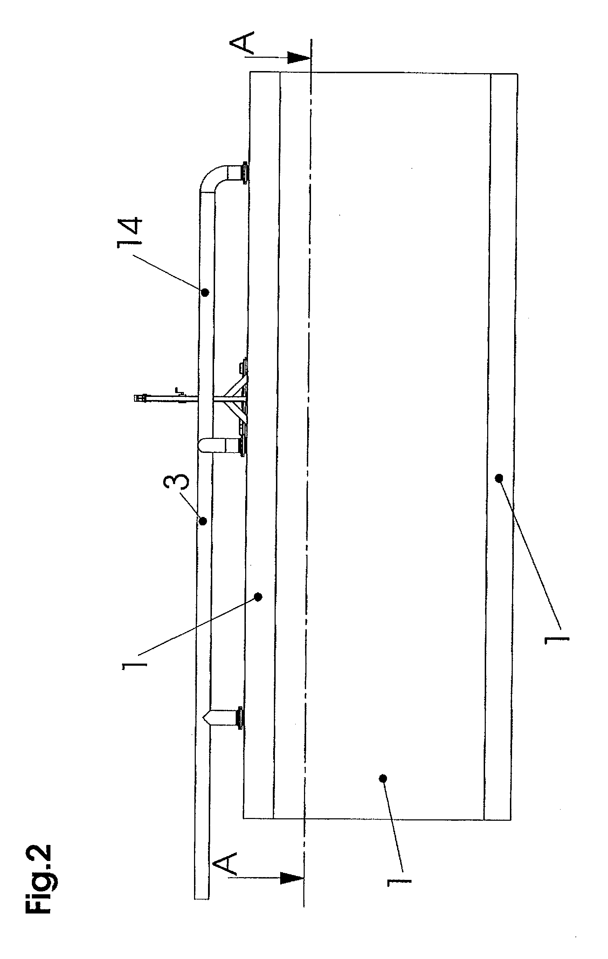

Fig.1 zeigt eine schematische Darstellung eines erfindungsgemäßen Gärbehälters 1 in Schnittdarstellung gemäß Linie AA aus Fig.2.

Ein auf mindestens 35°C beheizbarer Gärbehälter 1 ist in seinem Inneren mit Zwischenwänden versehen, die gemeinsam mit den den Gärbehälter 1 aufbauenden Wänden ein vorzugsweise mäanderförmiges Ringkanalsystem 5 ausbilden. Das Ringkanalsystem 5 weist einen in Bezug auf seine Gesamtlänge errechenbaren mittleren Strömungsquerschnitt auf, der abhängig ist von der Gesamtgeometrie des Ringkanalsystems 5. Diese kann beliebig ausgeführt sein und ist abhängig von diversen Randbedingungen, die an dieser Stelle nicht weiter ausgeführt werden sollen.

In das Ringkanalsystem 5 werden über eine nicht gezeichnete Zuleitung organische Einsatzstoffe 2 eingebracht, zum Zwecke der Gärung/Entgasung über einen bestimmten Zeitraum, vorzugsweise zwischen 30 und 60 Tagen. Der Gärbehälter 1 weist Mittel 14 (siehe Fig.2) zum Abtransport des durch die Gärung/Entgasung entstehenden Biogases 3 auf.

In einer geeigneten Position (Querschnitt) 8 des Ringkanalsystems 5 ist eine Schottwand 9 angeordnet, die an dieser Stelle den Querschnitt 8 des Ringkanalsystems 5 komplett ausfüllt und das Ringkanalsystem 5 teilt bzw. unterbricht. Die Schottwand 9 ist mit einer Öffnung 11 versehen, die ein Überströmen der Einsatzstoffe 2 von einem auf der einen Seite der Schottwand befindlichen Abschnitt des Ringkanalsystems 5 in einen Abschnitt auf der anderen Seite der Schottwand 9 ermöglicht. Die Öffnung 11 weist einen Umwälzquerschnitt 6 auf, durch welchen das im Gärbehälter befindliche Substrat im Ringkanalsystem 5 umgewälzt werden kann, wobei der Umwälzquerschnitt 6 erfindungsgemäß kleiner ist als der mittlere Strömungsquerschnitt des Ringkanalsystems 5.

Unter Umwälzquerschnitt 6 wird im vorliegenden Fall jener Querschnitt der in der Schottwand 9 vorhandenen Öffnung 11 verstanden, der im Betriebszustand der Anlage für das Überströmen der Einsatzstoffe vom Abschnitt des Ringkanalsystems 5 auf der einen Seite der Schottwand 9 in den Abschnitt des Ringkanalsystems 5 auf der anderen Seite der Schottwand 9 zur Verfügung steht.

Um die Überströmung zu bewerkstelligen, ist es erforderlich, die sich im Ringkanalsystem 5 des Gärbehälters 1 befindlichen Einsatzstoffe 2 umzuwälzen. Zu diesem Zweck ist eine Antriebseinheit 4 vorgesehen, die einen Rotor 10 aufweist, der in einem Strömungsring/-rohr 7 angeordnet ist.

Der Strömungsring/-rohr 7 ist im wesentlichen dicht an die Öffnung 11 angekoppelt, so dass die Antriebseinheit 4 die Einsatzstoffe 2 in Umwälzrichtung 15 strahlförmig durch den Umwälzquerschnitt 6 befördert.

Um das Überströmen der Einsatzstoffe 2 durch die Öffnung 11 in unterschiedlichen vertikalen Positionen des Gärbehälters 2 zu ermöglichen, ist es in besonders bevorzugten Ausführungsvariante der Erfindung vorgesehen, dass die Öffnung 11 entlang der Schottwand 9 zumindest vertikal verschiebbar angeordnet ist.

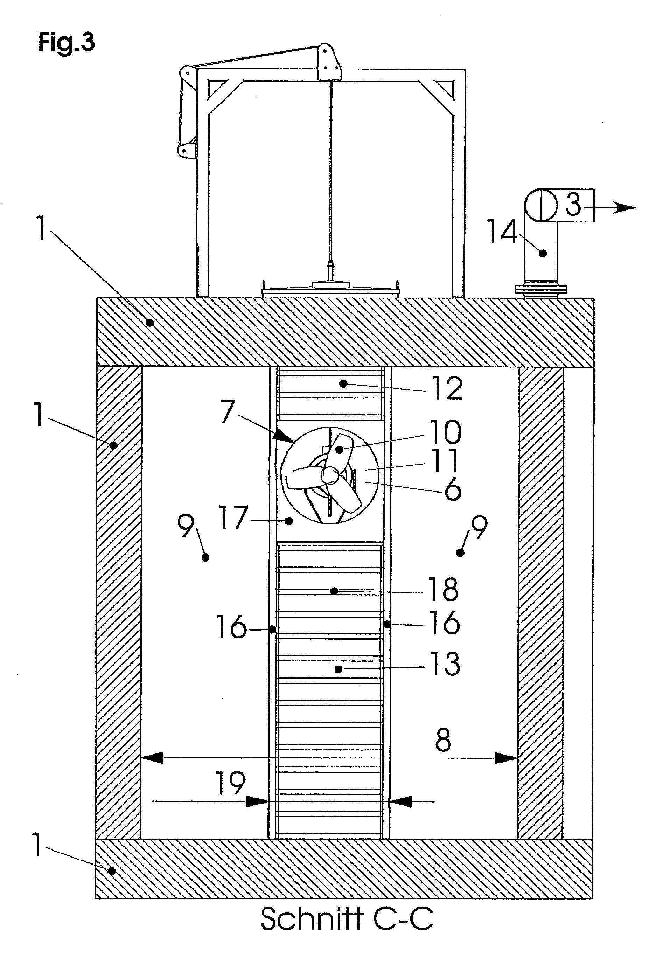

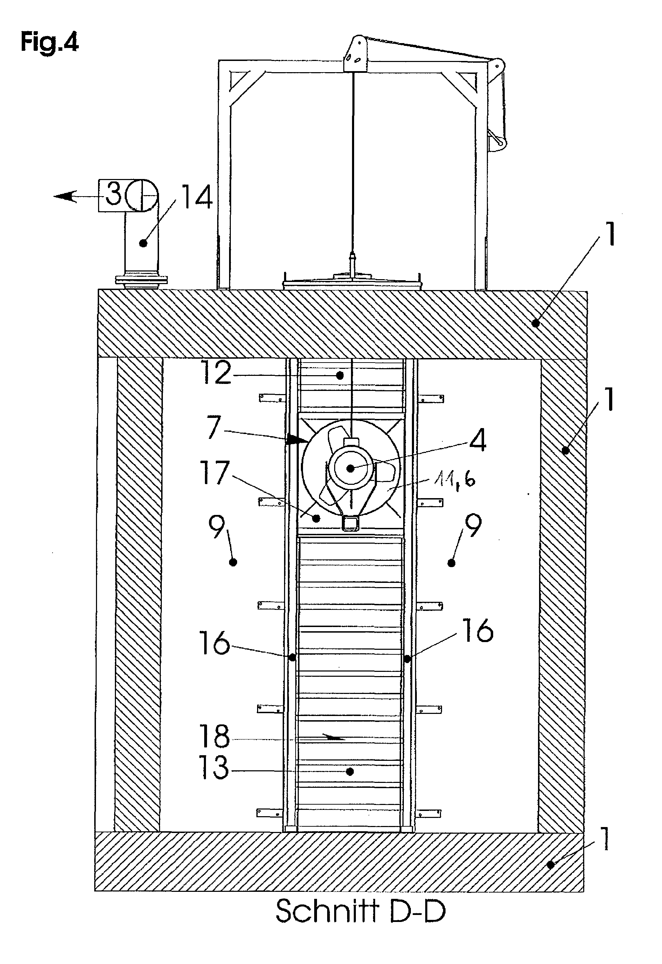

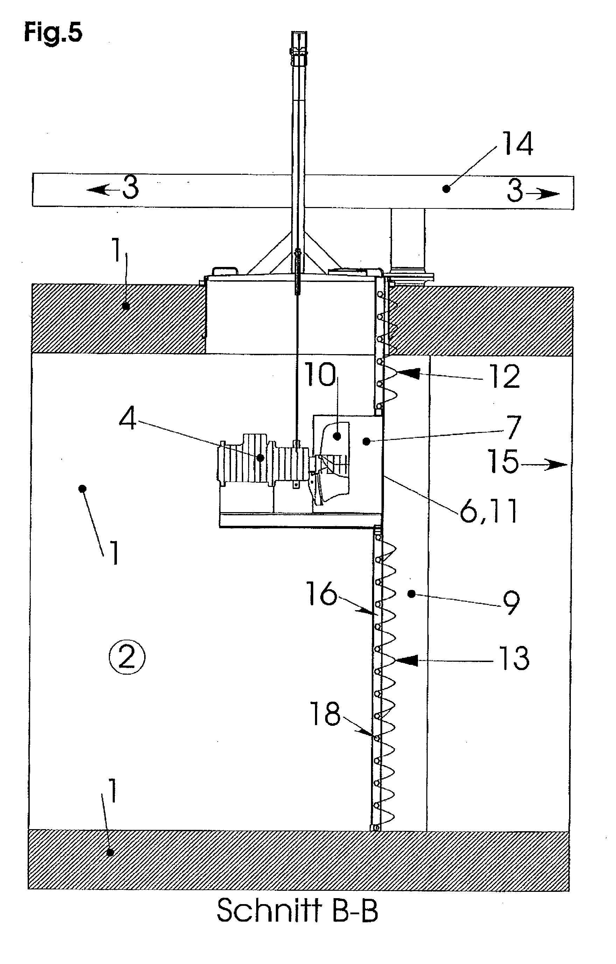

Dies kann beispielsweise dadurch erreicht werden, dass die Schottwand 9 einen sich über die gesamte bzw. einen Großteil der Höhe der Schottwand 9 (siehe Fig.3,4,5) verlaufenden Durchbruch 19 aufweist und an einer Seite der Schottwand 9, im Bereich der Kanten des Durchbruchs 19 ein Schienensystem 16 angeordnet ist, über welche die Ankoppelung des Strömungsrings/-rohres 7 an den Durchbruch 19 mittels einer Halterung 17 erfolgt.

Die Halterung 17 7 ist im Schienensystem 16 verschiebbar. Die verbleibenden Bereiche des Durchbruchs 19 oberhalb und unterhalb der Halterung 17 sind durch jalousie- oder vorhangartige, in ihrer Länge variable, beispielsweise zusammenschiebbare Elemente abgedeckt, so dass als Öffnung 11 in der Schottwand 9 jener Bereich zwischen unterster Kante des oberen jalousie- oder vorhangartigen Elementes und der oberen Kante des unteren jalousie- oder vorhangartigen Elementes fungiert. Der tatsächlich für die Umwälzung des Substrats zur Verfügung stehende Umwälzquerschnitt 6 ist in diesem Ausführungsbeispiel kleiner als die Öffnung 11.

In diesem Bereich ist der Strömungsring/-rohr 7 samt Halterung 17 an den Durchbruch 19 gekoppelt.

Auf diese Art und Weise kann der Strömungsring/-rohr 7 inkl. Halterung 17 und Antriebseinheit 4 entlang des Schienensystems 16 d.h. entlang des Durchbruchs 19 verfahren werden, wodurch sich eine entlang der Schottwand 9 verschiebbare Öffnung 11 ergibt.

Die damit verbundenen Leckagen im Bereich der jalousie- oder vorhangartigen Elemente beeinflussen das Ergebnis des erfindungsgemäßen Verfahrens bzw. die Funktionsweise des erfindungsgemäßen Gärbehälters nicht und können daher bei der weiteren Beschreibung der Erfindung außer Acht gelassen werden.

Typische Abmessungen eines erfindungsgemäßen Gärbehälters wären beispielsweise ein durchschnittlicher Strömungsquerschnitt des Ringkanalsystems von ca. 6 m² 2 bis 9 m² und eine Breite des Durchbruchs 19 von 500mm bis 1100mm sowie eine Umwälzquerschnitt 6 zwischen 0,3 m² und 0,6 m².

Im vorliegenden Ausführungsbeispiel kommen als jalousie- oder vorhangartige, in ihrer Länge variable Elemente flexible Schottvorhänge 12, 13 zum Einsatz, die aus gewebeverstärktem PVC-Planenmaterial gefertigt sind. Ein Schottvorhang 12 ist am oberen Ende der Halterung 17 sowie am oberen Ende des Durchbruchs 19 befestigt, der andere Schottvorhang 13 am unteren Ende der Halterung 17 sowie am unteren Ende des Durchbruchs 19. Über die Länge der Schottvorhänge 12,13 verteilt sind mit diesen verbundene und im Schienensystem 16 geführte Querstreben 18 angeordnet, wodurch Schottvorhänge 12,13 eine Mehrzahl an faltbaren bzw. zusammenschiebbaren Segmenten ausbilden.

Der Einsatz des erfindungsgemäßen Verfahrens mithilfe der erfindungsgemäßen Vorrichtung und die sich dadurch einstellende Zwangszirkulation der Einsatzstoffe 2 im Ringkanal 5 des Gärbehälters 1 ermöglicht den Verzicht auf den Einsatz von mehreren herkömmlichen Rührwerken zur lokal begrenzten Durchmischung der Einsatzstoffe 2 bei mesophil (35° -42°C) bzw. thermophil (42°-55°C) ablaufenden Verfahren zur Herstellung von Biogas.

Des weiteren ist es, wie in der vorliegenden Ausführungsvariante dargestellt, bei Biogasanlagen mit einem Volumen des Gärbehälters bis zu ca. 500m³ möglich, lediglich eine einzige Schottwand 9 samt Antriebseinheit 4 einzusetzen und dennoch eine für die Verhinderung von Sink- und Schwimmschichten ausreichende Zwangszirkulation zu erzeugen.

### BEZUGSZEICHENLISTE

- 1: Gärbehälter
- 2: organische Einsatzstoffe
- 3: Biogas
- 4: Antriebseinheit
- 5: Ringkanalsystem
- 6: Umwälzquerschnitt
- 7: Strömungsring/-rohr
- 8: ein Querschnitt des Ringkanalsystems
- 9: Schottwand
- 10: Rotor der Antriebseinheit
- 11: Öffnung in der Schottwand
- 12: oberer Schottvorhang
- 13: unterer Schottvorhang
- 14: Mittel zur Entnahme des Biogases
- 15: Umwälzrichtung der Einsatzstoffe
- 16: Schienensystem
- 17: Halterung
- 18: Querstreben
- 19: Durchbruch in der Schottwand

## Patentansprüche

1. Verfahren zur Erzeugung von Biogas (3) durch anaerobe Vergärung organischer Einsatzstoffe (2), welche in flüssiger und/oder fester Form in einen vorzugsweise beheizbaren Gärbehälter (1) eingebracht werden und in weiterer Folge in einen geschlossenen Nachgärbehälter bzw. ein Endlager für die Zwecke des weiteren Ausgärens umgelagert werden, **dadurch gekennzeichnet, dass** die organischen Einsatzstoffe (2) in einem einen mittleren Strömungsquerschnitt aufweisenden Ringkanalsystem (5) des Gärbehälters (1) umgewälzt werden, wobei die Umwälzung durch eine Antriebseinheit (4) bewirkt wird, welche die organischen Einsatzstoffe (2) durch einen gegenüber dem mittleren Strömungsquerschnitt des Ringkanalsystem (5) verringerten, betreffend seiner vertikalen Position im Gärbehälter einstellbaren Umwälzquerschnitt (6) einer Öffnung (11) befördert, welche in einer einen Querschnitt des Ringkanalsystems (5) ausfüllenden Schottwand (9) angeordnet ist, welche unterhalb und oberhalb der Öffnung (11)aus faltbaren, in ihrer Länge variablen Schottvorhängen (12,13) besteht.

2. Verfahren zur Erzeugung von Biogas (3) gemäß Anspruch 1, **dadurch gekennzeichnet, dass** die Umwälzung durch den Umwälzquerschnitt (6) in Zeittakten erfolgt.

3. Verfahren zur Erzeugung von Biogas nach Anspruch 1 oder 2, **dadurch gekennzeichnet, dass** die Einbringung der Einsatzstoffe (2) in den Gärbehälter (1) im Ansaugbereich der Antriebseinheit (4) erfolgt.

4. Gasdicht abgeschlossener, vorzugsweise beheizbarer Gärbehälter (1) zum Einsatz in einem Verfahren nach einem der Ansprüche 1 bis 3, **dadurch gekennzeichnet, dass** eine Antriebseinheit (4) zur Umwälzung der organischen Einsatzstoffe (2) in einem Ringkanalsystem des Gärbehälters (1) vorgesehen ist, wobei der Rotor (10) der Antriebseinheit von einem Strömungsring/-rohr (7) umgeben ist, welches an eine Öffnung (11) einer einen Querschnitt (8) des Ringkanalsystems (5) ausfüllenden Schottwand (9) angekoppelt ist und die Öffnung (11) einen Umwälzquerschnitt (6) aufweist, der kleiner ist als ein mittlerer Strömungsquerschnitt des Ringkanalsystems (5), wobei die Öffnung (11) entlang der Schottwand (9) vertikal, verschiebbar ist und die Schottwand (9) oberhalb und unterhalb der Öffnung (11) aus faltbaren, in ihrer Länge variablen Schottvorhängen (12,13) besteht.

5. Gasdicht abgeschlossener, vorzugsweise beheizbarer Gärbehälter (1) nach Anspruch 4, **dadurch gekennzeichnet, dass** das Ringkanalsystem (5) meanderförmig ausgebildet ist.

6. Gasdicht abgeschlossener, vorzugsweise beheizbarer Gärbehälter (1) nach einem der Ansprüche 4 bis 5, **dadurch gekennzeichnet, dass** im Gärbehälter (1) lediglich eine einzige Schottwand (9) samt Antriebseinheit (4) vorgesehen ist.

## Claims

1. A method for producing biogas (3) by anaerobic fermentation of organic starting materials (2), which are introduced in liquid/and/or solid form into a preferably heatable fermentor (1), and are subsequently retransferred to a closed secondary fermentor or a final storage unit for the purpose of final fermentation, **characterized in that** the organic starting materials (2) are circulated in a ring channel system (5) of the fermentor (1) having an average flow cross-section, wherein the circulation is produced by a drive unit (4) which conveys the organic starting materials (2) through a circulation cross-section (6) of an opening (11) which is reduced in comparison with the average flow cross-section of the ring channel system (5) and is adjustable with respect to its vertical position in the fermentor, and which is arranged in a partition wall (9) filling a cross-section of the ring channel system (5), which partition wall consists of foldable partition curtains (12, 13) of variable length beneath and above the opening (11).

2. A method for producing biogas (3) according to claim 1, **characterized in that** the circulation through the circulation cross-section (6) occurs in time cycles.

3. A method for producing biogas (3) according to claim 1 or 2, **characterized in that** the introduction of the starting materials (2) into the fermentor (1) occurs in the intake region of the drive unit (4).

4. A preferably heatable fermentor (1) which is sealed in a gastight manner for use in a method according to one of the claims 1 to 3, **characterized in that** a drive unit (4) is provided for circulating the organic starting materials (2) in a ring channel system of the fermentor (1), wherein the rotor (10) of the drive unit is enclosed by a flow ring/tube (7) which is coupled to an opening (11) of a partition wall (9) filling a cross-section (8) of the ring channel system (5) and the opening (11) has a circulation cross-section (6) which is smaller than an average flow cross-section of the ring channel system (5), wherein the opening (11) is vertically displaceable along the partition wall (9), and the partition wall (9) consists of foldable, partition curtains (12, 13) of variable length above and beneath the opening (11).

5. A preferably heatable fermentor (1), which is sealed in a gastight manner, according to claim 4, **characterized in that** the ring channel system (5) is arranged in a meandering fashion.

6. A preferably heatable fermentor (1), which is sealed in a gastight manner, according to one of the claims 4 to 5, **characterized in that** only one single partition wall (9) plus drive unit (4) is provided in the fermentor (1).

## Revendications

1. Procédé pour la production de biogaz (3) par fermentation anaérobie de matières premières organiques (2), qui sont introduites sous forme liquide et/ou solide dans une cuve de fermentation (1) pouvant de préférence être chauffée puis transférées dans une cuve de post-fermentation fermée ou un stockage final aux fins de poursuite de la fermentation, **caractérisé en ce que** les matières premières organiques (2) sont mises en circulation dans un circuit de conduites annulaires (5) de la cuve de fermentation (1) présentant une section d'écoulement moyenne, la circulation étant réalisée par une unité d'entraînement (4) qui achemine les matières premières organiques (2) à travers une section de circulation (6) d'une ouverture (11) réduite par rapport à la section d'écoulement moyenne du circuit de conduites annulaires (5) et réglable dans sa position verticale dans la cuve de fermentation, laquelle ouverture est disposée dans une cloison de séparation (9) remplissant une section du circuit de conduites annulaires (5), qui se compose en dessous et au-dessus de l'ouverture (11) de rideaux de séparation (12, 13) repliables et de longueur variable.

2. Procédé pour la production de biogaz (3) selon la revendication 1, **caractérisé en ce que** la circulation à travers la section de circulation (6) est réalisée de façon cadencée dans le temps.

3. Procédé pour la production de biogaz selon la revendication 1 ou 2, **caractérisé en ce que** l'introduction des matières premières (2) dans la cuve de fermentation (1) s'effectue dans la zone d'aspiration de l'unité d'entraînement (4).

4. Cuve de fermentation (1) fermée de manière étanche aux gaz et pouvant de préférence être chauffée, destinée à être utilisée dans un procédé selon l'une des revendications 1 à 3, **caractérisée en ce qu'**il est prévu une unité d'entraînement (4) pour la mise en circulation des matières premières organiques (2) dans un circuit de conduites annulaires de la cuve de fermentation (1), dans laquelle le rotor (10) de l'unité d'entraînement est entouré d'une anneau/tube d'écoulement (7) qui est couplé à une ouverture (11) d'une cloison de séparation (9) remplissant une section (8) du circuit de conduites annulaires (5) et l'ouverture (11) présente une section de circulation (6) qui est plus petite qu'une section d'écoulement moyenne du circuit de conduites annulaires (5), l'ouverture (11) pouvant être déplacée verticalement le long de la cloison de séparation (9) et la cloison de séparation (9) se composant au-dessus et en dessous de l'ouverture (11) de rideaux de séparation (12, 13) repliables et de longueur variable.

5. Cuve de fermentation (1) fermée de manière étanche aux gaz et pouvant de préférence être chauffée selon la revendication 4, **caractérisée en ce que** le circuit de conduites annulaires (5) est conformé en méandres.

6. Cuve de fermentation (1) fermée de manière étanche aux gaz et pouvant de préférence être chauffée selon l'une des revendications 4 à 5, **caractérisée en ce qu'**une seule cloison de séparation (9) avec une unité d'entraînement (4) est prévue dans la cuve de fermentation (1).
